Europäisches Patentamt

European Patent Office

Office européen des brevets

(11) Veröffentlichungsnummer : **0 150 248 B1**

(12) **EUROPÄISCHE PATENTSCHRIFT**

(45) Veröffentlichungstag der Patentschrift :
19.10.88

(51) Int. Cl.⁴ : **A 61 K 31/765**

(21) Anmeldenummer : 84101007.7

(22) Anmeldetag : 01.02.84

(54) **Mittel zur Behandlung und Prophylaxe uratischer und gemischter uratischer Lithiasis.**

(43) Veröffentlichungstag der Anmeldung :
07.08.85 Patentblatt 85/32

(45) Bekanntmachung des Hinweises auf die Patenterteilung : 19.10.88 Patentblatt 88/42

(84) Benannte Vertragsstaaten :
CH DE FR GB LI NL

(56) Entgegenhaltungen :
US-A- 4 143 130
JOURNAL OF THE AMERICAN PHARMACEUTICAL ASSOCIATION, Band XXXVIII, Nr. 8, August 1949, Seiten 428-432; S.S. HOPPER et al.: "Some toxicological properties of surface-active agents"

(73) Patentinhaber : T P O "PHARMACHIM"
Iliensko Chaussée 16
Sofia (BG)

(72) Erfinder : Georgieva, Maria Ivanova, Dr. med.
Kom. Manifest-Strasse Bl. 29-2
Sofia (BG)
Erfinder : Georgiev, Georgi Marinov
Kom. Manifest-Strasse Bl. 29-2
Sofia (BG)
Erfinder : Zonev, Dragomir Todorov
L. Stanev-Strasse 14
Sofia (BG)
Erfinder : Minkov, Evgeni Hristov
Komplex Mladost-2, Block 227-A, Eingang 2
Sofia (BG)
Erfinder : Iliev, Svetlin Tatchev, Dr. med.
Kompl. Lyulin, Bl.210
Sofia (BG)
Erfinder : Poneva, Miryana Vesselinova
D. Voynikov-Strasse 6
Sofia (BG)

(74) Vertreter : von Füner, Alexander, Dr. et al
Patentanwälte v. Füner, Ebbinghaus, Finck Mariahilfplatz 2 & 3
D-8000 München 90 (DE)

**Beschreibung**

Die Erfindung betrifft ein Mittel zur Behandlung und Prophylaxe der uratischen, uratisch-oxalatischen und uratischphosphatischen Lithiasis.

Die Nephrolithiasis (Nierensteinkrankheit) ist die am häufigsten vorkommende urologische Erkrankung. Der chemischen Zusammensetzung der Konkremente nach unterscheidet man oxalatische, uratische, zystinische und phosphatische Lithiase. Die Behandlung dieser Erkrankung erfolgt zur Zeit vorwiegend mittels eines chirurgischen Eingriffs. Es sind zwar Verfahren und Mittel für eine medikamentöse Behandlung (Litholyse) vorhanden, doch diese sind nicht zuverlässig und besitzen nicht immer den gewünschten Heileffekt.

Bekanntlich werden zur Behandlung der uratischen Lithiasis und der gemischten uratischen Lithiasis Mittel angewandt, die auf der Basis von Salzkomponenten, wie Citrate (Natrium und Kalium), Bikarbonate und Zitronensäure hergestellt werden. Solche Präparate sind z. B. unter den Handelsnamen « Magurlit » und « Soluran » bekannt. Zur Behandlung der erwähnten Lithiasis werden auch Kräuterextrakte enthaltende Mittel eingesetzt. So ein Mittel ist zum Beispiel das unter dem Handelsnamen « Uralit » bekannte Präparat. Die bekannten Mittel besitzen alle als Wirkungsbasis die Eigenschaft der Salzkomponenten, den pH-Wert des Harns bei Kranken bis zu 7 aufrechtzuerhalten, wodurch die Kristallisation der Harnsäure verhindert wird (Handbuch der Arzneimittel, herausgegeben v. « Medizina und Fiskultura », Sofia, 1977 ; Rote Liste, 1979).

Die Nachteile der bekannten Mittel sind folgende :

- es besteht die Möglichkeit, daß der pH-Wert des Harns den Wert 7 überschreitet, demzufolge sich Phosphate niederschlagen können, was zu einer Niereninfektion führen kann ;

- die Wirkung der angeführten Präparate tritt erst nach einer langen Anwendungszeit (einige Monate) ein ;

- es werden toxische Nebenwirkungen beobachtet ;

- sie können nicht bei infektiösen Lithiasisformen angewandt werden ;

- diese Mittel sind bei gemischten Formen der uratischen Lithiasis nicht wirksam.

Die Aufgabe der Erfindung ist, ein Mittel zur Behandlung und Prophylaxe uratischer und gemischter uratischer Lithiasis bereitzustellen, indem eine zuverlässige und eine kurzfristige Auflösung (Litholyse) der Konkremente erreicht wird.

Diese Aufgabe wird durch ein Mittel gelöst, das als aktive Komponente einen nichtionogenen oberflächenaktiven Stoff der allgemeinen Formel R—COO $(CH_2CH_2O)_n$H enthält, in der R ein Rest der Linolsäure, Linolensäure oder Oleinsäure bedeutet und n eine Zahl von 18 bis 40 ist.

Die Wirkung des erfindungsgemäßen Mittels erfolgt auf dem Prinzip der Bildung von komplexen, wasserlöslichen Verbindungen mit der Harnsäure (als aufbauende Komponente der uratischen Konkremente), indem es diese auflöst und die Harnsäure durch den Urin aus dem Organismus entfernt.

Die Vorteile des erfindungsgemäßen Mittels bestehen im folgenden :

- die Wirkung wird schnell erreicht (bis zu einem Monat), ohne daß Nebenwirkungen beobachtet werden ;

- Das Mittel kann auch bei infektiösen Formen der uratischen Lithiasis angewandt werden, sowie auch bei gemischten Formen (uratisch-oxalatische und uratisch-phosphatische) ;

- es verändert den pH-Wert des Harns nicht, wodurch auch keine Gefahr zur Abscheidung von Phosphaten besteht ;

- die Arbeitsfähigkeit der Erkrankten wird schnell wiederhergestellt.

Anhand des nachstehend angeführten Ausführungsbeispiels soll die Erfindung näher erläutert werden.

Beispiel

Das Mittel wird in einer Einzeldosis von 0,375 g im Durchschnitt 3 bis 4 Mal pro Tag peroral verabreicht. Die durchschnittliche Behandlungsdauer beträgt ein bis eineinhalb Monate. Das Mittel kann als Sirup, Tropfen oder in Gelatinekapseln formuliert werden. Zu diesem Zweck wird der Wirkstoff mit bekannten Hilfsstoffen unter Beibehaltung der oben angeführten wirksamen Dosis verarbeitet.

Um die Wirksamkeit des Mittels in vitro auf uratische Konkremente zu prüfen bzw. zu beweisen, wurden Experimente bei 37 °C bei statischen Bedingungen durchgeführt. Zu diesem Zweck wurden Proben von uratischen, uratisch-oxalatischen und uratisch-phosphatischen Konkrementen nach Masse und nach Harnsäuregehalt analysiert. Nach einem Verweilen in 2,5 %igen wäßrigen Lösungen des Mittels wurden die Konkremente erneut auf die oben erwähnten Kennwerte analysiert. Die Lösungen des erfindungsgemäßen Mittels wurden auf die der in gelöster Harnsäure analysiert. Die durchschnittliche Verminderung der Masse der Konkremente nach einer Verweilzeit von 48 Stunden im erfindungsgemäßen Mittel beträgt 20 %.

Um die Wirksamkeit des Mittels auf dieselben Komponenten, aber in einem Medium-Urin zu prüfen, bzw. zu beweisen, sind Experimente durchgeführt worden die zeigen, daß hier die durchschnittliche Verminderung der Masse der Konkremente für 48 Stunden 23 % beträgt. Um die Wirksamkeit der Lösungen des erfindungsgemäßen Mittels unter dynamischen Bedingungen zu beweisen, wurden Experimente in Apparaten durchgeführt, die dem menschlichen System der Harnorgane ähnlich konstruiert sind, bei welchem die durchschnittliche Verminderung der Masse der Konkremente auf 44,7 % anwächst. Es wurden auch Vergleichsversuche mit dem Präparat « Soluran » sowohl unter statischen als auch unter dynamischen Bedingungen durchgeführt. Die durchschnittliche Verminderung der Masse der uratischen Konkremente in statischen Verhältnissen des Solurans beträgt 7,1 %, während dieselbe bei dem erfindungsgemäßen Mittel 19,6 % beträgt. Unter dynamischen Bedingungen beträgt die Verminderung der Konkremente unter der Wirkung des Solurans 21,9 %, während dieselbe beim erfindungsgemäßen Mittel auf 44,7 % ansteigt.

Um den Wirkungsmechanismus des erfindungsgemäßen Mittels besser zu erläutern, wurden Ultraviolett-Spektren der Lösungen des Mittels vor und nach dem Verweilen uratischer Konkremente in diesen Lösungen aufgenommen. Es wurde festgestellt, daß das erfindungsgemäße Mittel ein charakteristisches Ultraviolett-Spektrum mit einem Maximum bei 262 nm, 272 nm und 284 nm aufweist. Bei einem Verweilen uratischer Konkremente in Lösungen des Mittels erscheint ein Peak der Harnsäure bei 286 nm. Der letztere zeigt im Vergleich mit dem Ultraviolett-Spektrum der Harnsäure in destilliertem Wasser eine Versetzung ihres charakteristischen Peaks von 290 nm auf 286 nm. Diese Versetzung in Richtung der kürzeren Längen zeigt, daß die Harnsäure in den Lösungen des Mittels nicht frei, sondern in Form einer Komplexverbindung vorhanden ist.

Die Prüfung der Toxizität des erfindungsgemäßen Mittels an Versuchstieren sowohl unter den Bedingungen eines akuten Tests, als auch eines chronischen Tests zeigen, daß die $LD_{50}$ bei peroraler Einnahme bei 3 680 mg/kg Gewicht liegt. Es wurde keine reizende Wirkung auf die Schleimhaut, auf das subkutane und Muskelgewebe beobachtet. Es wurden auch keine Veränderungen des Blutdrucks, der Anzahl der Erythrozyten und der Enzyme registriert. Die inneren Organe weisen ebenfalls keine Veränderungen auf.

Es wurde an Personen, die nicht an Nephrolithiasis leiden, experimentiert, um die Ausscheidung des Mittels durch die Nieren festzustellen. Der Urin dieser Versuchspersonen wurde vorher auf folgende Kennwerte geprüft : pH-Wert, Oberflächenspannung, Menge der Harnsäure. Danach wurde jeder Person je eine Einzeldosis des erfindungsgemäßen Mittels (0,375 g) in einem Suppenlöffel, Sirup (2,5 % an Wirkstoff) verabreicht. Von 20 Minuten bis zu einer Stunde nach der Einnahme eliminieren die getesteten Personen das Mittel durch den Urin, was mit Hilfe des Ultraviolett-Spektrum festgestellt wurde. Der Urin zeigt eine Verminderung der Oberflächenspannung mit 0,0065 N/m (6,5 dyn/cm) im Durchschnitt, der pH-Wert verändert sich nicht, die Harnsäure bleibt konstant, doch in einer Komplexverbindung gebunden.

Es wurden Experimente an einer Gruppe Kranker, die an uratischer und gemischter uratischer Lithiasis leiden, durchgeführt. Diese Kranken wurden mit dem erfindungsgemäßen Mittel in Form eines Sirups behandelt, je ein Suppenlöffel drei Mal pro Tag eine Stunde vor dem Essen. Danach wurde das Blut und der Urin der Kranken analysiert sowie auch Röntgenaufnahmen und Echographien gemacht. Eine Litholyse (Auflösung) der Konkremente wurde in zwanzig Tagen bis zwei Monaten je nach der Größe der Konkremente erreicht. Die Arbeitsfähigkeit der Kranken wurde noch in der ersten Woche nach der Behandlung verbessert.

## Patentanspruch

Mittel zur Behandlung und Prophylaxe uratischer und gemischter uratischer Lithiasis, dadurch gekennzeichnet, daß es als Wirkstoff einen nichtionogenen oberflächenaktiven Stoff der allgemeinen Formel $RCOO(CH_2CH_2O)_nH$ enthält, in der R ein Rest der Linolsäure, Linolensäure oder Oleinsäure bedeutet und n eine Zahl von 18 bis 40 ist.

## Claim

Agent for the treatment and prophylaxis of uratic and mixed uratic lithiasis, characterised in that it contains as active substance a nonionic surface active substance of the general formula $RCOO(CH_2CH_2O)_nH$, in which R denotes a residue from linoleic acid, linolenic acid or oleic acid and n is a number of 18 to 40.

## Revendication

Agent pour le traitement et la prophylaxie de la lithiase uratique et uratique mixte, caractérisé par le fait qu'il contient, en tant que substance active, une substance tensio-active non-ionique représentée par la formule générale $RCOO(CH_2CH_2O)_nH$ dans laquelle R représente un reste d'acide linoléique, d'acide linolénique ou d'acide oléique ; et n est un nombre allant de 18 à 40.